(19) Europäisches Patentamt
European Patent Office
Office européen des brevets

(11) Publication number: 0 177 344 B1

(12) # EUROPEAN PATENT SPECIFICATION

(45) Date of publication of patent specification: **08.04.92**

(51) Int. Cl.[5]: **G01N 33/44**, //G01N30/00

(21) Application number: **85307045.6**

(22) Date of filing: **02.10.85**

(54) **Serum pretreatment for tricyclic antidepressant drug assays and kit therefor.**

(30) Priority: **02.10.84 US 657319**

(43) Date of publication of application:
**09.04.86 Bulletin 86/15**

(45) Publication of the grant of the patent:
**08.04.92 Bulletin 92/15**

(84) Designated Contracting States:
**AT BE CH DE FR GB IT LI LU NL SE**

(56) References cited:
**EP-A- 0 168 968**
**US-A- 4 056 468**
**US-A- 4 307 245**
**US-A- 4 460 695**

**TRENDS IN ANALYTICAL CHEMISTRY, vol. 3, no. 7, August 1984, pages 178-181, Amsterdam, NL; R.V. SMITH "Determination of drugs and metabolites in biological fluids"**

**Journal of Chromatographic Science, 18, (1980), 512-514**

(73) Proprietor: **SYNTEX (U.S.A.) INC.**
**3401 Hillview Avenue P.O. Box 10850**
**Palo Alto California 94303(US)**

(72) Inventor: **Collins, Christine Glen**
**3435 Kaylene Drive**
**San Jose California 95127(US)**
Inventor: **Pankay, Susan**
**2460 West Bayshore Road 6**
**Palo Alto California 94303(US)**
Inventor: **Jaklitsch, Anna**
**1850 Hamilton Avenue**
**Palo Alto California 94303(US)**

(74) Representative: **Armitage, Ian Michael et al**
**MEWBURN ELLIS & CO. 2/3 Cursitor Street**
**London EC4A 1BO(GB)**

EP 0 177 344 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

A number of tricyclic compounds find use in the treatment of depression. These tricyclic antidepressants include imipramine, desmethylimipramine (desipramine), amitriptyline, nortriptyline, protriptyline, doxepin and desmethyldoxepin (nordoxepin). In administering a tricyclic antidepressant, it is frequently necessary to ensure that the blood level of the antidepressant remains within a certain narrow concentration range in order to ensure effective dosage, while avoiding levels which may be toxic or produce undesirable effects. Furthermore, it is often necessary to detect potentially toxic levels of tricyclic antidepressants and their metabolites.

It is therefore desirable to provide a simple and rapid procedure for determining or detecting the levels of tricylic antidepressants in serum or other physiological fluids. The procedure should provide reproducible values and be specific for the tricyclic compounds which are measured. Thus, the procedure must be capable of distinguishing the tricyclic antidepressants from other drugs and from metabolites of the tricyclic antidepressant drug, which would otherwise give an erroneous result in an assay for the detection of tricyclic antidepressants.

Since the therapeutic range of the tricylic antidepressant drugs is from about 50 to 300 ng/ml, it is necessary not only to measure extremely small amounts of the tricyclic antidepressant drug in serum, but also to be able to distinguish between small differences in concentrations. Naturally occurring materials or metabolites of the tricyclic antidepressant drug in the serum sample may modify the observed signal so as to give falsely high results. It would therefore be desirable to provide for a simple means for pretreatment of a serum sample for a tricyclic antidepressant drug assay. The pretreatment method should be rapid and efficient and provide an assay sample containing the drug substantially free of interfering substances.

The tricyclic antidepressants are closely related chemically to one another. Techniques reported for the determination of amitriptyline in biological fluids include the use of thin layer chromatography, gas-liquid chromatography and GLC-mass spectrometry. Gifford, et al., J. of Chrom., 105, 107-113 (1975); Gupta, et al., Clin. Biochem., 9, 247-51 (1976); Nyberg and Martensson, J. Chromatography, 143, 491 (1977); Watson and Stewart, J Chrom., 134, 182 (1977); ibid. 132 155-159 (1977). Radioimmunnoassay has been reported for amitriptyline by Aherne, et al., Br. J. Clin. Pharmac., 3, 561 (1976), Turner, Lancet, 180, 1316 (1977); and Aherne, et al., Lancet 1214 (1977). In Aherne, et al., ibid., a synthesis for an antigen for use as an immunogen for antibody formation is described, where nortriptyline is substituted with aminobutylene followed by conjugation to bovine serum albumin employing carbodiimide. In another antigen conjugate synthesis by Kaul, et al., J. Anal. Tox., 1, 236 (1977), nortriptyline was conjugated to bovine serum albumin through a succinyl group. The resulting antibodies were found to have significant cross-reactivity with a number of other tricyclic drugs.

U.S. Patent No. 4,275,160 describes imipramine derivatives and poly(amino acid) conjugates. U.S. Patents Nos. 4,223,013 and 4,307,245 disclose amitriptyline conjugates to antigenic proteins and enzymes.

N-(2-carboxyethyl) derivatives of nortriptyline and desipramine are disclosed by Hubbard et al., J. Pharm. Sc., 67, pp. 1571-1578 (1978) and by Hubbard et al., Canadian Journal of Pharmaceutical Sciences, 15, pp. 89-93 (1980).

In one aspect of the present invention; serum samples for tricyclic antidepressant drug assays are pretreated by passing the serum sample through a column containing silica gel that is alkylated. After application of the serum sample, the column is washed with a wash mixture comprising from about 15 to 50 volume percent of an organic solvent containing from 1 to 6 carbon atoms and from 1 to 5 heteroatoms selected from oxygen, nitrogen, and sulfur and from about 50 to 85 volume percent of an aqueous buffered solution having a pH of from about 3.5 to 5.0. The drug is eluted from the column with an eluent comprising from about 25 to 100 volume percent of an organic solvent of from 1 to 6 carbon atoms and from 1 to 5 heteroatoms selected from oxygen, nitrogen, and sulfur and from about 0 to 75 volume percent of an aqueous buffered solution having a pH of about from 6 to 8. The eluted material contains the tricyclic antidepressant drug substantially free of interfering substances. The pretreatment method finds particular application in conjunction with assays employing enzyme or fluorescent labels.

### Description of preferred embodiments

Blood serum or plasma samples for tricyclic antidepressant drug assays are pretreated to provide a sample substantially free from metabolites present in the serum sample and in a form useful for a tricyclic antidepressant drug assay determination. By the term "substantially free" is meant that the tricyclic antidepressant drug sample contains less than about from 20%, preferably less than about from 10%, of metabolites of such drug.

The method normally employs liquid chromatography using a column containing silica gel alkylated with alkyl groups of from 1 to 12 carbon atoms, preferably 1 to 6 carbon atoms. The silica gel particles have a size in the range of about 30-50 µm, preferably about 40 µm. The particles are suitably silanized with methyl or ethyl silyl groups to provide the alkylated silica gel particles.

The amount of the column packing, i.e., alkylated silica gel, employed and the dimensions of the column are dependent on the size of the serum sample to be treated. Generally, for a serum sample of 0.5 ml, about 80 to 120 mg, preferably 90 to 110 mg, of packing is used. For 100 mg of silica gel, depending upon the manner of packing, as well as the diameter of the column, the height of the column can vary from about 6 to 10 mm.

The column is packed by introducing the silica gel powder into an appropriate column. The column is then conditioned, by adding an alcohol, such as methanol, and removing the alcohol by any convenient means, e.g., vacuum, positive pressure or centrifugation. After the alcohol has been removed, the column is then washed with water, preferably deionized water. The water is then removed as described above, and the column is now ready for the sample.

Prior to applying the sample to the column, the serum sample may be subjected to other protreatments. Depending upon the nature of the sample, the sample may, for example, be centrifuged.

The sample is then added to the column after all the excess methanol and water employed in the pretreatment and washing of the column have been removed. The sample may then be drawn into the column by vacuum or centrifugation, or it may be pushed into the column by positive pressure. The conditions for applying the sample to the column will be generally mild, for example, a vacuum in the range of about 250-500mm Hg (about 10-20 inches Hg) may be used. Various conventional devices can be used, for example, the Vac-Elut (trade mark) vacuum box (Analytichem International).

After the sample has been applied to the column, the column is washed with a mixture comprising a water soluble organic solvent and an aqueous buffered medium. The organic solvent has from 1 to 6, preferably 1 to 3, carbon atoms and from 1 to 5, preferably 1 to 2, heteroatoms selected from the group consisting of oxygen, nitrogen and sulfur. The organic solvent may, for example, be an alkylnitrile such as acetonitrile, propionitrile, an alcohol such as methanol, ethanol, propanol, or a ketone such as acetone. The wash mixture generally contains from about 15 to 50, preferably 25 to 35, volume percent of the organic solvent.

The wash mixture also contains from about 50 to 85, preferably 65 to 75, volume percent of an aqueous buffered medium having a pH of from about 3.5 to 5.0, preferably from about 4.0 to 4.4. The buffered medium may be about 0.1 to 1 M, preferably 0.2 to 0.4 M, in a metal salt of a carboxylic acid having from 2 to 4 carbon atoms. Exemplary of such metals are alkali metals such as sodium, potassium, and the like and exemplary of carboxylic acids are acetic acid and propionic acid.

The aqueous buffered medium portion of the column wash mixture may also contain an alkyl sulfonate wherein the alkyl group has from 5 to 7 carbon atoms such as, for example, pentane sulfonate, hexane sulfonate and heptane sulfonate. Conveniently, the alkyl sulfonate is incorporated into the mixture as part of the aqueous buffered medium. In this mode, the aqueous buffered medium would be from about 0 to 0.010 M in alkyl sulfonate.

The volume of the wash mixture should be sufficient to remove substantially all of the metabolites of the tricyclic antidepressant drug from the column. However, the wash mixture should remove substantially none of the drug itself. The volume of the wash solution is based primarily on the number of theoretical plates of the column packing. As an example, the volume of wash mixture can be from about 0.8 to 1.0 ml for about 100 mg of column packing. After addition of the wash solution, the wash solution may be drawn through the column as described above for the sample. Usually this will involve, for an initial volume of 1 ml sample, at least about 15 seconds and not more than about two minutes, generally from about 20 seconds to 45 seconds. Any water remaining at the tip of the column may be removed by blotting or other convenient means.

The tricyclic antidepressant drug is then eluted to provide for a tricyclic antidepressant drug sample substantially free from metabolites to be used in an assay. To this end an elution mixture is employed comprising one or more water soluble organic solvents and an aqueous buffered medium having a pH of from about 6 to 8, preferably, 6.5 to 7.5, more preferably neutral pH. The organic solvent comprises from about 25 to 100, preferably 70 to 80, volume percent of the elution mixture and the aqueous buffered medium usually comprises from about 0 to 75, preferably 20 to 30, volume percent of the elution mixture. Generally, the organic solvent has the same characteristics as those described above for the wash mixture and, conveniently, may be the same organic solvent as that employed in the wash mixture. Preferably, the elution mixture comprises at least two organic solvents as defined above in a ratio of about from 1:1 to 1:2. A preferred elution mixture may contain from about 45 to 55 volume percent of an alkylnitrile as defined

above and from about 25 to 35 volume percent of an alcohol as defined above for the organic solvent.

The aqueous buffered medium may comprise a phosphate buffer such as potassium hydrogen phosphate. The phosphate may conveniently be combined with the water prior to combination with the other eluent components. In this embodiment, the aqueous medium is usually from 0 to 0.01 M in phosphate buffer.

Elution is accomplished by adding from about 0.5 to 1.0 ml of the eluent mixture for an initial volume of 1 ml of serum sample. Generally, the volume of eluent mixture should be sufficient to remove substantially all of the drug from the column; usually the volume corresponds to the initial volume of the serum sample. The eluent is drawn through the column in the same manner described above for the wash mixture. The eluate is then collected and is ready to be used in an assay since it contains the tricyclic antidepressant drug substantially free of interfering substances, i.e., those substances which, by their presence in the sample, would affect the accuracy of the assay.

It is also within the scope of the present invention to dilute the eluate prior to conducting an assay. Generally, about 0.5 to 1 ml of an aqueous buffer of pH 5 to 8, such as Tris HCl buffer is passed through the column from which the sample was eluted. The buffer is then combined with the eluted sample.

In assays involving labels, e.g., enzyme labels, the components of the wash mixture and of the eluant should have little or no detrimental effect on the label activity.

Tricyclic antidepressant drugs, for which samples treatable by the method of the present invention are assayed, include derivatives of dibenzazepine, dibenzocycloheptadiene, and dibenzoxepin, generally of the following formula:

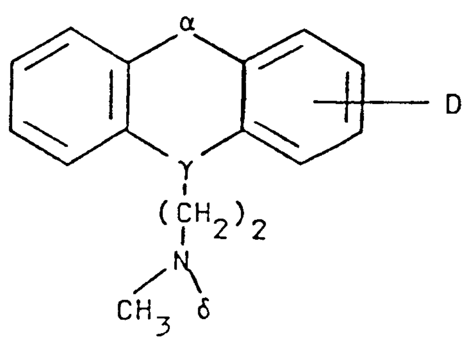

(Ia)

wherein:

α is $CH_2$-$CH_2$, $CH_2$-CH(OH), CH=CH, or $CH_2$-O;

Y iS N-$CH_2$, C=CH, or N-CH(R) wherein R is alkyl of 1 to 3 carbon atoms, particularly $CH_3$;

δ is H or $CH_3$; and

D is hydrogen, hydroxy, or a halogen atom of atomic number 9 to 53, preferably 7 to 35, more preferably a chlorine atom.

Exemplary of such tricyclic antidepressant compounds are imipramine, desmethylimipramine, amitriptyline, protriptyline, trimipramine, chlomipramine, doxepin and desmethyldoxepin. Also nortriptyline.

Serum samples treated to give tricyclic antidepressant drug extracts in accordance with the present invention may be assayed for the presence of the drug by a number of assay methodologies. The assays may be heterogeneous or homogeneous involving labels such as enzymes, radioisotopes and fluorescers.

The invention also includes a kit comprising, in a packaged combination, (1) a prepacked column typically having dimensions as described above and containing silica gel alkylated, suitably with alkyl groups containing from 1 to 12 carbon atoms and in amounts as described above, to which column the serum sample is to be applied, (2) suitably from 100 to 200 ml of a wash mixture as described above, and (3) suitably from 100 to 200 ml of an element as described above. The wash mixture and the eluent may be in suitable containers such as vials made of a suitable material such as glass or plastic. The kit may also include ancillary items such as a device for securing the serum sample or applying the serum sample to the column, column conditioning solutions as described above, wash solutions such as an alcohol, deionized water, 100 to 200 ml of a post elution aqueous buffer as described above, in separate containers. The above kit may be combined with an assay kit for performing a tricyclic antidepressant drug assay or it may be separate therefrom.

EXAMPLE

The invention is further demonstrated by the following illustrative example.

EXAMPLE 1

Assay for Nortriptyline

A 100 mg C-2 (ethyl) column from Analytichem International was washed with approximately one ml of methanol followed by approximately one ml of water. The sample (500 µl) was placed on the top of the column. A vacuum apparatus was attached to the bottom and a vacuum was drawn on the column. The eluate obtained was discarded and the column was washed with 900 µl of a solution which was 70% 0.4 M sodium acetate, 5 mM heptane sulfonate, pH 4.2, and 30% acetonitrile. A vacuum was again drawn on the column and the eluate was discarded. Next, the column was contacted with 500 µl of a solution which was 50% acetonitrile, 25% methanol, and 25% 5 mM $K_2HPO_4$, pH 7. The eluant was collected and used in the assay procedure.

An enzyme conjugate and an antibody reagent were prepared in accordance with the teaching of European patent application no.: 85303601.0. [The method used was as set out in Preparation A, following.]

In carrying out the assay, a Gilford Stasar III (trade mark) microsample spectrophotometer was employed with a Thermocuvette with a flow cell. All readings were made at 340 mn. The following solutions were prepared as reagents for use in the assay.

Buffer:
0.055 M tris-HCl pH 8.1 (RT)

Enzyme Conjugate Reagent:

Buffer
0.9% NaCl
1.0% β-lactoglobulin (BLG), pH 8.0 (RT)
Sufficient enzyme conjugate to give a maximum rate of ΔOD equal to 700-1200 in the assay medium

Assay buffer:

Buffer
0.5% NaCl
0.01% (v/v Triton X-100, pH 8.0 (RT)

Antibody Reagent:

Buffer
0.1% BLG,
G-6-P(Na) 0.198 M,
Nicotine adenine dinucleotide (NAD) 0.12 M, pH 5.2 (RT)
Antinortriptyline optimized for assay (antibodies prepared in sheep). All % indicated are w/v, g/100 ml.

The protocol employed for carrying out an assay was as follows:

Into a diluter was drawn 15 microliters (µl) of the above sample. The sample was dispensed with 250 microliters of the assay buffer into a one milliliter Croan cup followed by 15 µl of the antibody reagent with 250 µl of the assay buffer. After 50 sec. incubation 15 µl of the enzyme reagent and 250 µl of the assay buffer were added. Immediately after the enzyme addition, the entire sample was aspirated into the flow cell. After 10 seconds, a first reading was taken, followed by a second reading after a 50 second interval. The results are reported as the difference in absorbance X 2.667.

| Sample Concentrating of Nortriptyline (ng/ml). | ΔOD |
|---|---|
| 0 | 695* |
| 25 | 723 |
| 50 | 761 |
| 100 | 807 |
| 175 | 850 |
| 250 | 870 |

* Lowest rate in assay with predetermined amount of antibody.

EXAMPLE 2

Assay for Desmethylimipramine

A 100 mg column (C-2 (ethyl) from Analytichem, Harbor City, California) was washed with approximately one ml of methanol followed by approximately one ml of water. The sample (500 µl) was placed on the top of the column. A vacuum apparatus was attached to the bottom and a vacuum was drawn on the column. The eluate obtained was discarded and the column was washed with 900 µl of a solution which was 70% 0.4 M sodium acetate and 5 mM heptane sulfonate, pH 4.2, 30% acetonitrile. A vacuum was again drawn on the column and the eluate was discarded. Next, the column was contacted with 500 µl of a solution which was 50% acetonitrile, 25% methanol, and 25% 5 mM $K_2HPO_4$, pH 7. The eluant was collected and used in the assay procedure.

The antibodies and the enzyme conjugate employed in this assay for desmethylimipramine were prepared as set out in Preparation B, following.]

In carrying out the assay, a Gilford Stasar III® microsample spectrophotometer was employed with a Thermocuvette (3017T) with a flow cell. All readings were made at 340 nm. The following solutions were prepared as reagents for use in the assay.

Buffer:

0.055 M tris-HCl pH 8.0 (RT)

Enzyme Conjugate Reagent:

Buffer
0.9% NaCl
1.0% BLG, pH 8.0 (RT)
Sufficient enzyme conjugate to give a maximum rate of ΔOD equal to 800-1200 in the assay medium

Assay buffer:

Buffer
0.5% NaCl
0.01% (v/v) Triton X-100, pH 8.0 (RT)

Antibody Reagent:

Buffer
0.1% BLG,
G-6-P(Na) 0.22 M,
NAD 0.13 M, pH 5.2 (RT).
Antidesmethylimipramine optimized for assay (antibodies were prepared in sheep)
(All % indicated are w/v, g/100 ml.)

The protocol employed for carrying out an assay was as follows:

Into a diluter was drawn 15 microliters (µl) of the above eluant. This sample was dispensed with 250

microliters of the assay buffer into a one milliliter Croan cup followed by 15 µl of the antibody reagent with 250 µl of the assay buffer. After 50 sec. incubation 15 µl of the enzyme reagent and 250 µl of the assay buffer were added. Immediately after the enzyme addition, the entire sample was aspirated into the flow cell. After 10 seconds, a first reading is taken, followed by a second reading, after a 50 second interval. The results are reported as the difference in absorbance x 2.667.

| Sample Concentration of Desmethylimipramine (no/ml) | ΔOD |
|---|---|
| 0 | 702* |
| 50 | 737 |
| 100 | 765 |
| 200 | 800 |
| 350 | 834 |
| 500 | 854 |

* lowest rate in assay with predetermined amount of antibody.

The assay of Example 1 was repeated except that the serum sample was not pretreated in accordance with the present invention. The following represents a statistical summary of the results where the y-axis represents the enzyme-label assay result and the x-axis represents a reference method conducted using high pressure liquid chromatography (HPLC).

| Nortriptyline | | |
|---|---|---|
| | Pretreated Sample | Non-pretreated Sample |
| n | 14 | 12* |
| slope | 1.11 | 2.36 |
| intercept | 10.39 | 87.46 |
| correlation | .98 | .83 |
| SEE | 9.2 | 26.7 |

* Two samples had rates higher than the highest calibrator and therefore could not be accurately quantitated.

It is evident from the above results that the subject method provides a sample allowing for an accurate assay for tricyclic antidepressant drugs, particularly involving enzyme labels. Thus, a sensitive and efficient method is provided for treating samples for tricyclic antidepressant drug assays which results in accurate determinations of the drug.

PREPARATION A, FOR EXAMPLE 1

EXAMPLE A

Preparation of 3-nitrodibenzosuberone

Acetic anhydride (15 ml) was added slowly to white fuming nitric acid (90%, 6.1 ml, 0.13 mole) at room temperature. The resulting warm (30°) solution was cooled to 25° and was added dropwise to a solution of dibenzosuberone (20.8 g, 0.1 mole, from Aldrich Chemical Co.) in 25 ml of acetic anhydride at room temperature for a period of three hours. After addition, an aliquot was withdrawn, and quenched in water, and partitioned in dichloromethane; tlc showed the presence of 3-nitrodibenzosuberone, some fast moving substance, and starting material. The reaction mixture was then added to 2 liters of ice water and the oily product was stirred for half an hour. The resulting aqueous layer was decanted and discarded, and the oily residue on the bottom was dissolved in dichloromethane and washed with saturated sodium bicarbonate and brine. The organic layer was dried over $MgSO_4$ and evaporated to yield light yellow oil which was crystallized by dissolving in warm ether and adding hexane until cloudiness developed. The resulting clear solution was cooled (5°) overnight to yield 6.5 g of pale yellow solid (26% yield) of 3-nitrodibenzosuberone. The tlc of this material showed a major product and a small amount of impurity; the product was used

without further purification.

| Anal. Calcd. for $C_{15}H_{11}NO_3$, | | | |
|---|---|---|---|
| | C, 71.15; | H, 4.35; | N, 5.53 |
| Found | C, 69.42; | H, 4.34; | N, 5.85 |

EXAMPLE B

Preparation of 3-aminodibenzosuberone

To a suspension of 3-nitrodibenzosuberone (27.2 g, 0.107 mole) from Example A in a mixture of tetrahydrofuran (800 ml), isopropanol (800 ml) and phosphate buffer [pH 6.5, 1.6 liters, prepared by mixing 13.6 g $KH_2PO_4$ (0.1 molar) and 27.8 ml of IN NaOH and diluting the resulting solution to 2 liters] was added sodium dithionite (220 g, from Eastman Organic Chemicals) over a period of 5 min. The solids became soluble, and after 15 min. tlc showed complete reaction. The resulting clear solution was then extracted exhaustively with ethyl acetate and the organic layer was washed with saturated NaCl solution, and dried over $Na_2SO_4$. Evaporation of solvents gave yellow crude product which was chromatographed on a silica gel column and eluted with ether/hexane 1:1 to yield 8.7 g (36% yield) pure yellow 3-aminodibenzosuberone.

EXAMPLE C

Preparation of 3-amino-5-(3-dimethylaminopropyl)-5-hydroxy-10,11-dihydrodibenzo[b,e]cycloheptatriene

a. Preparation of N,N-dimethylpropylchloride.

A solution of N,N-dimethylpropyl chloride hydrochloride (100 g, from Aldrich Chemical Co.) in about 100 ml water was made alkaline by adding 10% NaOH to pH of about 11 - 12. The resulting bi-layer solution was then extracted with ether, and the ether extracts were dried over $MgSO_4$. Then, the ether was distilled using a simple distillation apparatus under 1 atm pressure, and the resulting liquid was distilled at 45° (60 mm pressure) to yield 54.5 g colorless liquid of N,N-dimethylpropyl chloride.

b. Grignard reaction:

To Mg turnings (13 g., 0.54 mole) in tetrahydrofuran (53 ml, dried and distilled freshly over the sodium salt of benzophenone) was added a few drops of 1,2-dibromoethane (J. T. Baker Chemical Co.) under nitrogen. After crushing the turnings with a glass rod and noting gas evolution, a solution of N,N-dimethylpropyl chloride (32.3 g, 0.27 mole) in tetrahydrofuran (150 ml) was added dropwise. During this addition the solution was heated sufficiently to maintain a gentle reflux. After stirring for 1 h under reflux, the brown reaction mixture was cooled to room temperature followed by addition of 3-aminodibenzosuberone (12.4 g, 0.056 mole) from Example B in dry THF (220 ml). The resulting brown product was allowed to stir at room temperature for half an hour and was cautiously quenched with saturated ammonium chloride (200 ml). The resulting yellow residue was extracted with ethyl acetate. The organic phase was washed with saturated $NaHCO_3$ and brine, dried ($Na_2SO_4$), and evaporated to yield a yellow oil (17.2 g) of 3-amino-5-(3-dimethylaminopropyl) 5-hydroxy-10,11-dihydrodibenzo[b,e]cycloheptatriene.

EXAMPLE D

Preparation of 3-aminoamitriptyline

To a solution of 3-amino-5-(3-dimethylaminopropyl)-5-hydroxy-10,11-dihydrodibenzo[b,e] cycloheptatriene (17.1 g, 0.055 mole) from Example C in dichloromethane (300 ml) was added trifluoroacetic acid (29.6 ml, 0.175 mole). The resulting dark brown solution was refluxed for 18 hours; tlc of an aliquot of the reaction mixture showed incomplete reaction. Therefore, p-toluenesulfonic acid monohydrate (10.5 g, 0.055 mole) was added and refluxed overnight. After 18 hours, complete reaction was observed. The reaction product was cooled, diluted with ether, made alkaline with concentrated ammonia (22 ml), and then extracted with ethyl acetate. Evaporation of solvents gave a foaming product containing two major components and some impurities of higher Rf values. The samples were purified using preparative HPLC (silica gel column, $NH_3$:MeOH:$CH_2Cl_2$/0.24:3:97 by volume):

Fractions were collected and analyzed using both refractive index and analytical tlc (silica gel plate, 0.08:1:7/$NH_3$:MeOH:$CH_2Cl_2$). Fractions of the same Rf values were combined and evaporated to give 6.8 g cis-3-aminoamitriptyline Rf 0.08 and 3.6 g trans 3-aminoamitriptyline Rf 0.15. Total yield of the products from 3-aminodibenzosuberone over two steps is 10.4 g (64% yield). Anal. calcd. for cis 3-aminoamitriptyline, $C_{20}H_{24}N_2$ 1/2$H_2O$, C, 79.73; H, 8.31; N, 9.30. Found, C, 79.53; H, 7.99; N, 8.89.

EXAMPLE E

Preparation of cis -N'-(methyldithioacetyl)-3-aminoamitriptyline

To a solution of cis-3-aminoamitriptyline (2.7 g, 9.3 mmole) from Example D in a mixture of tetrahydrofuran (80 ml, dried and distilled freshly from sodium benzophenolate) and dichloromethane (20 ml, dried over molecular sieves 3A) was added the NHS ester of methyldithioacetic acid (2.9 g, 13.8 mmole). The resulting solution was allowed to stir at room temperature. After four days, complete reaction was observed on tlc. The resulting light yellow solution was evaporated to dryness on a rotary evaporator and the residue was chromatographed on a reversed phase silica gel column [containing 300 g silica gel 60 silanized], and eluted with 3% MeOH/$CH_2Cl_2$ (1.4 liter) and then 5% MeOH/$CH_2Cl_2$ (600 ml). After evaporation of solvents, 4.3 g light yellow foaming product, which contained the cis-N'-methyl(dithioacetyl)3-aminoamitriptyline was obtained. The product was dissolved in 100 ml of $CH_2Cl_2$, and 1 ml triethylamine was added. The organic layer was washed with saturated NaCl and dried over $Na_2SO_4$, and evaporated to yield 3.3 9 (86% yield) foaming product of cis-N'-methyl(dithioacetyl)3-aminoamitriptyline.

EXAMPLE F

Preparation of cis-N($\beta$,$\beta$,$\beta$-trichloroethoxycarbonyl)-N-methyl(dithioacetyl)-3-aminonortriptyline

To a solution of cis-N'-(dithioacetyl)-3-aminoamitriptyline (3 g, 7 mmole) from Example E in dichloromethane (120 ml, dried over molecular sieves 3A) was added trichloethyl chloroformate (9.6 ml, 70 mmoles) dropwise at room temperature under nitrogen atmosphere followed by triethylamine (9.7 ml, 70 mmoles) for a period of 15 min. The slightly warm reaction mixture was cooled using a water bath and then allowed to stand at room temperature for 3.5 h. Complete reaction was obtained as observed on analytical silica gel plates. The resulting yellow solution was evaporated to dryness. Ether (100 ml) was added; white precipitates which formed were filtered and then washed with ether. The ether filtrates were collected and evaporated to yield a brown oil which was chromatographed on silica gel. Fractions were analyzed by tlc and detected using both UV and $I_2$ since the product showed a UV chromophore, while the impurities had no UV absorption but showed brown spots of higher Rf values when being developed in an $I_2$ chamber. Fractions were combined to give (3.2 g, 77% yield) cis-N-($\beta$,$\beta$,$\beta$-trichloroethoxycarbonyl) 3-amino-N'-(methyldithioacetyl) nortriptyline as a white foaming product. Fractions containing impurities were either chromatographed again or discarded since the impurities interfered with the reductive cleavage of the methyldithioacetyl derivative in the next reaction. The sample after column chromatography showed the correct structure.

| Anal. Calcd. for $C_{25}H_{27}N_2O_3Cl_3S_2$ | | | | | |
|---|---|---|---|---|---|
| | C, 52.31; | H, 4.71; | N, 4.88; | Cl, 18.57 | S, 11.16. |
| Found: | C, 52,41; | H, 4.85; | N, 4.73; | Cl, 18.14; | S, 10.84. |

EXAMPLE G

Preparation of cis-3-amino-N'-(mercaptoacetyl) nortriptyline

All solutions used in work up of the sulfhydryl derivative were degassed by bubbling argon through each solution at room temperature for at least 10 min.

To a solution of cis-N-($\beta$,$\beta$,$\beta$,-trichloroethoxycarbonyl)-3-amino-N'-(methyldithioacetyl) nortriptyline (500 mg, 0.87 mmole) from Example F in glacial acetic acid (10 ml) was added activated zinc dust (1.5 g) at room temperature under nitrogen. The zinc dust was activated by washing well with 100 ml of 2% HCl for 4-5 min, then filtered and zinc powder was washed with water, ethyl alcohol, acetone and dry ether. The

powder was then dried overnight at reduced pressure at room temperature and then used for reduction. The reaction mixture was allowed to stir overnight at room temperature. After 22 hours, the reaction mixture was filtered and washed with about 40 ml of water, and the filtrate was cooled in an ice bath. White precipitates (Rf 0.95, 114 mg) formed and were removed by filtration and discarded, and the filtrate was extracted with 2 x 10 ml ether/hexane (1:1), or until the complete removal of side product. The resulting aqueous solution was then extracted with a total of 200 ml dichloromethane and the organic solution washed with brine and dried over $Na_2SO_4$. Evaporation of solvents gave a clear viscous oil of the acetate of cis 3-amino-N'-(mercaptoacetyl) nortriptyline (104 mg, 29% yield, Rf 0.31).

The product was found to be decomposed under vacuo at room temperature within a day. However, under acidic conditions, e.g., the acetate salt, the product was found to be more stable. The acetate salt of 3-amino-N'-(mercaptoacetyl) nortriptyline was stored under nitrogen, or argon, and kept at dry ice temperature.

## EXAMPLE H

### Preparation of the Conjugate of Cis-3-amino-N'-(mercaptoacetyl) Nortriptyline and Bromoacetylglycyl BgG

a. Preparation of the NHS ester of bromoacetylglycine

To a solution of bromoacetylglycine (1 g, mp. 114-115°) in 10 ml of DMF was added powdered NHS (1 g) and EDCI (1 g, 5.2 mmole) under nitrogen at 0°. The resulting clear solution was then allowed to stir at 5° after 18 h and used directly without the isolation of the NHS ester.

b. Conjugation of bromoacetylglycine to BgG

To a clear solution of BgG (1.5 g) in a mixture of phosphate buffer (100 ml, pH 9, 0.05 M) and DMF (5 ml) was added dropwise the NHS ester of bromoacetylglycine (500 mg in 6 ml DMF, prepared as above) at 0° for a period of 30 min. The pH of the BgG solution before the addition of NHS solution was 8. The pH dropped to 6.3 after addition of the NHS solution; the pH was then adjusted to 6.8. The resulting mixture was allowed to stir overnight at 5°. After 18 hours, the conjugate was dialyzed against 4 x 4 liter phosphate buffer (0.0125 M, pH 6.8) 2 x 4 liter (0.05 M, pH 6.8). The conjugate was diluted to 150 ml and stored for further conjugation. The concentration of this protein conjugate was determined by UV and found to be 9.58 mg/ml.

c. Conjugation of cis-3-amino-N'-(mercaptoacetyl) nortriptyline to bromoacetylglycyl BgG

To the bromoacetylglycyl BgG solution (212 mg) prepared as above in 30 ml of 0.1 m phosphate buffer (pH 7, pre-degassed with nitrogen) was added cis-3-amino-N'-(mercaptoacetyl) nortriptyline acetate (35 mg in 1.75 ml DMF) prepared as in Example G. The resulting cloudy solution was kept under nitrogen at 5° for 70 hours. The milky solution was then dialyzed against 2 x 4 liter $NH_4OH$-$H_2O$, pH 9, 2x1 liter 8M urea, 1 liter 4M urea, 1 liter 2M urea and then 5x4 liter $NH_4OH$-$H_2O$, pH 9. The pH of the conjugate was adjusted to 10 with 15% $NH_4OH$ and centrifuged at 3K, 10 min. Supernatant was lyophilized to give a conjugate (205 mg) of hapten number 46.

## EXAMPLE I

### Preparation of the Conjugate of Cis-3-amino-N'-(mercaptoacetyl) Nortriptyline and Bromoacetylglycyl BSA

a. Preparation of conjugate of bromoacetylglycine to BSA

To a clear solution of BSA (1.5 g) in phosphate buffer (pH 9.0, 0.05 M, 100 ml) and DMF (6 ml) was added dropwise the NHS ester of bromacetylglycine (500 mg), in 6 ml DMF at 0° for a period of 30 min. Before the addition of the NHS ester, the pH of the BSA solution was about 8.0. After the addition of the NHS ester, the pH dropped to 5-6; the pH (5.86) of the reaction mixture was adjusted to 6.8 and stirred overnight at 5°. The resulting conjugate was then dialyzed against 3 x 4 liter phosphate buffer (0.0125M, pH 6.8) and 2 x 4 liter phosphate buffer (0.05 M, pH 6.8). The conjugate was diluted to 150 ml and stored for further conjugation. The concentration of this protein conjugate was determined by UV and found to be 8.8 mg protein/ml solution.

b. Conjugation of cis-3-amino-N'-(mercaptoacetyl) nortriptyline to bromoacetylglycyl BSA

To the bromoacetylglycyl BSA (250 mg) prepared as above in a mixture of phosphate buffer (41 ml. pH 7) and DMF (8 ml), [the solutions used for conjugation were saturated with nitrogen gas] was added cis-3-amino-N'-(mercaptoacetyl) nortriptyline free base (65 mg, prepared as in Example G) in 3 ml of DMF. The resulting mixture was then stirred under nitrogen at 5° for a total of 72 hours and dialyzed exhaustively against $NH_4OH/H_2O$ (10 x 4 l). The conjugate was then lyophilized to give 194 mg protein

(hapten number 24).

EXAMPLE J

Preparation of the Conjugate of trans-3-Amino-N'-(mercaptoacetyl)nortriptyline and Bromacetylglycyl G-6-PDH

a) Preparation of conjugate of bromoacetylglycine (BAG) and G-6-PDH.

The above BAG/G-6-PDH conjugate was prepared according to the procedure disclosed in U.S. Patent No. 4,220,722 at columns 18-19 (the disclosure of which is incorporated herein by reference). The conjugate was dialyzed against 4 l of tris buffer without preservatives (.05% azide .005% Thimerasol) to give 13.8 mg of conjugate in 6.1 ml.

b) Conjugation of trans-3-amino-N'-(mercaptoacetyl)nortriptyline to bromoacetylglycyl G-6-PDH.

A solution of 19 mg trans-3-amino-N'-(mercaptoacetyl) nortriptyline from Example G in 0.5 ml of DMF containing 50 µl of glacial acetic acid was prepared. This solution (125 µl) was degassed with argon and was added to the 6.1 ml of the dialyzed BAG/G-6-PDH from above. The resulting mixture was stirred for 3.5 h at 4° and then centrifuged. The supernatant was chromatographed on a Sephadex G-50 column and fractions containing protein were collected. The product was 84% deactivated and 40% inhibitable.

EXAMPLE K

Preparation of the Conjugate of G-6-PDH and 5-(3-N-Methylaminopropyl)-10,11-dihydro-10-thioacetamido-5H-dibenz[b,f]azepine

a. Preparation of the conjugate of bromoacetyl glycine and G-6-PDH (61 mg) with 8 ml of 0.055 M tris buffer at pH 3.0) was brought to 4° and 320 mg each of G-6-P($Na_2$) salt and NADH were added and dissolved. To this solution a 0.5 M bromoacetylglycyl NHS ester in DMF, prepared as in Example J above, was added slowly with stirring until the deactivation of the enzyme was 65%. The solution was dialyzed against tris buffer (0.055 M, pH 8.0, 4000 ml) for 18 h.

b. Conjugation of 5-(3-N-methylaminopropyl)-10,11-dihydro-10-thioacetamido-5H-dibenz[b,f]azepine to bromoacetylglycyl G-6-PDH.

The hapten material (63 mg) from Example E was reconstituted in 1.5 ml DMF. All of the dialyzed material from Example I was placed inside a flask and cooled to 4°. The hapten was added dropwise until the inhibition against anti-DMI antibodies was 45-50% (a hapten to enzyme ratio of about 95). The G-6-PDH conjugate was then desalted at 4° over a G50 column with tris buffer (.055 M, pH 8.0) with preservatives.

EXAMPLE L

Antibodies were produced in conventional manner in response to the conjugate of Example H, and used in the assay together with the enzyme conjugates prepared in Example K.

PREPARATION B, FOR EXAMPLE 2

Example M

Preparation of 5-[3-N-methyl-N-(2,2,2,-trichlorocarboethoxy)amino propyl]-10,11-dihydro-5H-dibenz [b,f] azepinone

Into a 200 ml round bottom flask was placed 8.0 g (0.25 mol) of 5-(3-dimethylaminopropyl)-5H-dibenz [b,f] azepin-10-one, (prepared according to the teaching of U.S. Patent No. 4,275,160, Examples 1-5), 45 ml of anhydrous toluene, 14 g (0.102 mol) of anhydrous potassium carbonate followed 21.7 g (0.102 mol) of 2,2,2-trichloroethyl chloroformate. The solution was stirred vigorously and slowly brought to reflux with an oil bath. Reflux continued for approximately 12 hours. TLC analysis, silica gel, GF-chloroform showed some carbamate formed after less than one hour's time (visualization with UV lamp and ceric sulfate-$H_2SO_4$ spray). The reaction was cooled and added to a separatory funnel containing 200 ml water and was extracted several times with chloroform. The organic phase was again extracted with two 100-ml portions of

10% HCl, 200 ml water, dried over $MgSO_4$, filtered, and concentrated on a rotary vaporator leaving a dark brown oil (~15 g).

Chromatography of the oil was performed on a glass column with dry silica gel 60-200 mesh (J. T. Baker Chemical, Phillipsburg, N.J.) ~650 g, 5X80 cm column dimension. The crude product, dissolved in a small amount of dichloromethane, was placed at the top of the column. Two liters of solvent (dichloromethane) was collected prior to collection of ~20 ml increments on an automatic fraction collector. After faster moving impurities were eluted, larger volume fractions were collected and TLC indicated only one spot corresponding to carbamate. These fractions were combined and concentrated to give 9.8 g of product.

IR: $CDCl_3$ (1670 $cm^{-1}$, aromatic carbony]); (1720 $cm^{-1}$ carbamate carbonyl stretch)
Pmr: 90MHz($CDCl_3$-TMS) δ8.1 (dd,l 1H, J~9Hz,
J~1.5Hz, 1H aromatic); 7.13 (m, 7H aromatic);
4.64 (br.s, 2H,-$OCH_2CCl_3$); 3.96 (s,2H,-$CH_2CO$-);
3.96 (t,2H,J~6Hz,-N-$CH_2CH_2$-);
3.34 (t,2H,J~Hz,-$CH_2CH_2NCH_3$);
2.85 (s,3H,-$NCH_3$); 1.89 (m,2H,$CH_2$,$CH_2$-$CH_2$)

Example N

Preparation of 5-[3 N-methyl-N-(2,2,2-trichlorocarboethoxy)amino propyl)-10-amino-10,11-dihydro-5H-dibenz [b,f]azepine

Into a 100 ml pear-shaped flask equipped with a $CaCl_2$ drying tube and a reflux condenser was placed 3.8 g (0.00835 mol) of the product of Example M, 6.4 g (0.0835 mol) of anhydrous ammonium acetate (dried in dessicator under vacuum over $CaCl_2$), 50 ml of an anhydrous methanol and 1.5 g (0.0250 mol) of sodium cyanotrihydridoborate. The mixture was stirred and brought to 50°.

After 72 h, an additional 1 g of anhydrous ammonium acetate and 5 gm sodium cyanotrihydridoborate was added.

After 100 h the reaction mixture cooled and was poured into a separatory funnel containing 300 ml water and was extracted with three 100-ml portions of chloroform. The organic phases were combined, dried with $MgSO_4$, filtered and concentrated on a rotary vaporator.

One-half of the material was applied to twelve 20X20 cm preparative plates, 2.5 mm silica gel 60 PF-254+366 (Analtech). The plates were treated with ethyl ether 3 times. Material was then extracted from the absorbant with 10% methanol-90% dichloromethane and concentrated. This material was re-subjected to the above chromatographic condition, but ethyl ether saturated with ammonia gas was substituted for the ethyl ether and the plates were treated only one time. (The major band was collected after UV light inspection indicated the presence of the desired material.) These two chromatographic steps gave pure product which was isolated in 60-70% yield.

IR: $CHCl_3$ (1720 $cm^{-1}$, carbamylcarbonyl);
(3380-3350 $cm^{-1}$, NH stretching modes)
Pmr: 90MHz ($CDCl_3$-TMS) δ7.07 (m,8H,aromatic);
5.64 (S,2H,O-$CH_2CCl_3$);
4.48 (m,1H,-$NH_2$-CH-$CH_2$);
3.75 (t,2H,J~6Hz,N-$CH_2$-$CH_2$);
3.34 (t,2H,J~6Hz;
2.85 (s,3H,-$NCH_3$);
2.13 (br.s,2H,$NH_2$)

Example O

Preparation of N-hydroxysuccinimidylmethyldithioacetic acid

Into a 50 ml round bottom flask was placed 1.2 g (8.86 mmols) of methyl dithioacetic acid (prepared according to P. Singh, et al. (1979) Anal. Biochemistry 104, 51) 35 ml of dichloromethane, and 1.09 g (9.54 mmols) of NHS (crystallized from ethyl acetate). The solution was cooled in an ice bath before addition of 1.96 g (9.54 mmols) of distilled N,N'-dicyclohexylcarbodiimide; a mild exothermic reaction occurred. After stirring for 4 h, the solution was filtered through a medium sintered glass funnel, precipitated urea was washed with dichloromethane and light brown filtrate concentrated on a rotary evaporation at ambient

temperature.

This material was dissolved in 10 ml $CH_2Cl_2$ and applied to the top of a 2.5 × 43 cm dry glass column packed with 110 g of silanized silica gel 60 particle size 0.063 - 0.200 mm (70-230 mesh ASTM, E Merck).

The eluant was l/l-$CH_2Cl_2$/hexane. The fractions were monitored by tlc on silanized silica gel RP-2 (E. Merck) with l/l-$CH_2Cl_2$/hexane as the eluant.

Fractions 22-40 were combined (dry weight 1.2 g, approx. 60% yield). The dry material was dissolved in $CH_2Cl_2$/hexane solution and cooled overnight at 0°. More hexane was added as needed to promote crystallization (900 g white NHS ester, m.p. 79-81°).

Microanalysis sulfur calc. 27.25%; Act. 27.24%.

## Example P

### Preparation of 5-(3-N-methyl-N-(2,2,2-trichlorocarboethoxy)aminopropyl)-10,11-dihydro-10-methylditioacetamido-5H-dibenz [b,f] azepine

A solution of 557 mg (1.22 mmols) of 5-[3-N-methyl-N-(2,2,2-trichlorocarboethoxy)amino propyl]-10-amino-10,11-dihydro-5H-dibenz [b,f] azepine from Example 2, 287 mg (1.22 mmols) of N-hydroxysuccinimidyl methyl dithioacetate from Example O and 25 ml of anhydrous tetrahydrofuran was stirred at ambient temperatures for 4 h.

TLC analysis was conducted on silanized silica gel RP-2 eluant 20% hexane-80% dichloromethane; comparison with N-hydroxysuccinimidyl methyldithioacetate, N-hydroxysuccinimide and starting amine revealed that the reaction was complete. (Rf value of product ~0.13.)

The reaction mixture was concentrated and dissolved in 20% hexane-80% $CH_2CL_2$ and placed at the top of a glass column 2.5x42 cm dry packed with 130 gm of silica gel 60, silanized particle size 0.063-0.20 mm (70-230 mesh ASTM) RP-2. The eluant was 20% hexane-80% dichloromethane and 15-20 ml fractions were collected (approximately 96% yield).

IR: 1% in KBr (1650 $cm^{-1}$ amide carbonyl);
(1740 $cm^{-1}$ carbamate carbonyl)

Pmr: 90 MHz ($CDCl_3$-TMS) $\delta$2.4 (s,3H,$SCH_3$);
2.88 (br.s,3H,-$NCH_3$) 3.38 (s,2H,-$COCH_2S$-);
4.68 (br.s,2H,-O-$CH_2CCl_3$);
5.62 (br.m, 1H,-CH-NHCO-)

## Example Q

### Preparation of 5-(3-N-methylaminopropyl)-10,11-dihydro-10-thioacetamido-5H-dibenz [b,f] azepine

Into a 25 ml round bottom flask equipped with stopper was placed 140 mg (0.243 mmol) of the product of Example P, excess zinc dust (>70 mg), 10 ml of glacial acetic acid. The mixture was stirred at ambient temperatures for 24 h (a white precipitate of $ZnCl_2$ formed). Reaction progress was monitored by TLC on RP-2 silanized silia gel plates with 15% methanol-85% dichloromethane as the eluant (the plates were observed under UV lamp and with Ellman's reagent spray.

The reaction mixture was filtered to remove solids, was washed with acetic acid-methanol, and was concentrated on a rotary vaporator under high vacuum without heating. The residue was taken up in water/chloroform extracted with many portions of chloroform dried with $MgSO_4$, filtered, and concentrated. This material was applied to one preparative TLC 20x20 cm plate, 2.5 mm thickness, silanized silica gel PF 254 (E. Merck). The plate was eluted with 15% mechanol-85% dichloromethane. The band corresponding to the desired product was isolated. The product was extracted from the above solvent with 20/80 methanol/dichloromethane, concentrated on rotary evaporator and under high vacuum giving a light yellow foam, 60 mg, 70% yield.

Rf value ~.62 sulfhydryl 85% $CH_2Cl_2$-15% methanol.

IR: $CHCl_3$ film (1650 $cm^{-1}$ amide carbonyl); (acetate salt -$NH_2^+$ $CH_3AcO^-$, 1720 $cm^{-1}$ $CO_2$-)

PMR: 90MHz ($CD_3OD/CDCl_3$-TMS) $\delta$7.1 (m,8H,aromatic);
$\delta$5.54 (m,1H,-CH-NCO-); 3.87(t,2H,J~6Hz,N-$\underline{CH_2}CH_2$-);
3.23 (s,-$CH_2SH$);
3.05 (t,2H,J~6Hz,-$CH_2CH_2$-$NHCH_3$); 2.60 (s,3H,$NCH_3$)
2.04 (m,2H,-$\underline{CH_2}CH_2$-$NHCH_3$)

Example R

Preparation of the Conjugate of BSA-bromoacetylglycine with 5-(3-N-methylaminopropyl)-10,11-dihydro-10-thioacetamido-5H-dibenz[b,f]azepine

a. Preparation of the NHS succinimic ester of bromoacetylglycine

To a solution of bromoacetylglycine (1 g, m.p. 114-115°) in 10 ml of DMF was added powdered N-hydroxysuccinimide (1 g) and EDCI (1 g, 5.2 mmole) under nitrogen at 0°. The resulting clear solution was then allowed to stir at 5° after 18 h and was used directly without the isolation of the NHS ester.

b. Preparation of the conjugate of bromoacetylglycine and BSA

To a clear solution of BSA (1.5 g) in phosphate buffer (pH 9.0, 0.05 M, 100 ml) and DMF (6 ml) was added dropwise the NHS ester of bromoacetylglycine (500 mg) prepared in (a) above, in 6 ml DMF at 0° for a period of 30 min. Before the addition of the NHS ester, the pH of the BSA solution was about 8.0. After the addition of the NHS ester, the pH dropped to 5-6, the pH (5.86) of the reaction mixture was adjusted to 6.8 and the mixture was stirred overnight at 5°. The resulting conjugate was then dialyzed against 3 x 4 liter phosphate buffer (0.0125M, pH 6.8) and 2 x 4 liter phosphate buffer (0.05 M, pH 6.8). The conjugate was diluted to 150 ml and stored for further conjugation. The concentration of this protein conjugate was determined by UV and found to be 8.8 mg protein/ml solution.

c. Conjugation of BSA-bromoacetylglycine and 5-(3-N-methylaminopropyl)-10,11-dihydro-10-thioacetamido-5H-dibenz[b,f]azepine

Fifty ml of the BSA-bromoacetylglycine solution from (b) above (~0.375 g) was placed in a 125 ml flask followed by 10 ml of .4M $Na_2HPO_4$-$NaH_2PO_4$ buffer pH 7.25 (pH of protein solution 7.21 by pH meter).

The solution was cooled in an ice bath at 4°; then 100 mg of the mercaptoacetamido product of Example Q was dissolved in 1 ml of DMF and was added slowly dropwise to the stirring protein solution. An additional 2 ml of DMF was used to rinse residual material. After the addition was complete, the protein solution appeared considerably turbid.

The above solution was stirred in a cold room (4°) for 3 days.

The above solution was placed in a semi-permeable membrane (cylinder diameter 20.4 mm, M.W. cut off 6,000-8,000) and dialyzed against deionized water pH 9.8 with $NH_4OH$, 4 liters, 3 times for 8 h each.

The material was then chromatographed using Sephadex (trade mark) G-50 medium with a bed volume of four times the volume of the product solution. Fractions of approximately 15 ml each were collected, UV of fractions was recorded on a Carey 15 spectrophotometer. The appropriate fractions were combined based on the UV data. The combined fractions were lyophilized to give 0.440 g of product with a hapten number of 24.

Example S

Preparation of the Conjugate of BgG-bromoacetylglycine with 5-(3-N-methylaminoproplyl)-10,11-dihydro-10-thioacetamido-5H-dibenz[b,f]azepine

a. Conjugation of bromoacetylglycine to BgG

To a clear solution of BgG (1.5 g) in a mixture of phosphate buffer (100 ml, pH 9, 0.05 M) and DMF (5 ml) was added dropwise the NHS ester of bromoacetylglycine (500 mg in 6 ml DMF, prepared as in Example Ra above, at 0° for a period of 30 min. The pH of the BgG solution before the addition of NHS solution was 8. The pH dropped to 6.3 after addition of the NHS solution; the pH was then adjusted to 6.8. The resulting mixture was allowed to stir overnight at 5°. After 18 hours, the conjugate was dialyzed against 4 x 4 liter phosphate buffer (0.0125 M, pH 6.8) 2 x 4 liter (0.05 M, pH 6.8). The conjugate was diluted to 150 ml and stored for further conjugation. The concentration of this protein conjugate was determined by UV and found to be 9.58 mg/ml.

b. Conjugation of BgG-bromoacetylglycine and 5-(3-N-methylaminopropyl)-10,11-dihydro-10-thioacetamido-5H -dibenz[b,f]azepine

Fifty ml of the BgG-bromoacetylglycine solution from (a) above (~0.375 g) was placed in a 125 ml flask and 10 ml of 0.4M $Na_2HPO_4$-$NaH_2PO_4$ buffer pH 7.23 was added. The mixture was then cooled to 4° in an ice bath. Next, was added 100 mg (0.281 mmol) of 5-(3-N-methylaminopropyl)-10,11-dihydro-10-thioacetamido-5H -dibenz[b,f]azepine from Example Q dissolved in 1 ml of DMF. The solution became turbid then very milky. An additional 2 ml of DMF used to rinse the remaining material into the

reaction vessel.

The above solution was stirred in a cold room (4°) for 3 days.

The above solution was placed in a semi-permeable membrane tubing (cylinder diameter 20.4 mm, M.W. cut off 6,000-8,000) and dialyzed against deionized water pH 9.8 with $NH_4OH$, 4 liters, 3 times for 8 h each.

The material was then chromatographed using Sephadex G-50 medium with a bed volume of four times the volume of the product solution. Fractions of approximately 15 ml each were collected, UV of fractions was recorded on Carey 15 spectrophotometer. The appropriate fractions were combined based on the UV data. The combined fractions were lyophilized to give 0.440 g of product with a hapten number of 8.

Example T

Preparation of the Conjugate of G-6-PDH and 5-(3-N-methylaminopropyl)-10,11-dihydro-10-thioacetamido-5H-dibenz[b,f]azepine

a. Preparation of the conjugate of bromoacetyl glycine and G-6-PDH

G-6-PDH (61 mg in 8 ml of 0.055 M Tris buffer at pH 8.0) was brought to 4° and 320 mg each G-G-P ($Na_2$) salt and NADH were added and dissolved. To this solution, a 0.5 M bromoacetylglycyl NHS ester in DMF, prepared as in Example Ra above, was added until the ester to enzyme ratio was 3:1 and the deactivation of the enzyme was 65%. The solution was dialysed against Tris buffer (0.055 M, pH 8.0) 4000 ml for 18 h.

b. Conjugation of 5-(3-N-methylaminopropyl) 10,11-dihydro-10-thioacetamido-5H-dibenz[b,f] azepine to bromoacetylglycyl G-6-PDH

The hapten material (63 mg) from Example Q was reconstituted in 1.5 ml of DMF. All of the dialysed material from Example Ta was placed in a side arm flask and cooled to 4°. The hapten was added dropwise until the inhibition against anti-DMI antibodies was 45-50% (a hapten to enzyme ratio of about 95). The G-6-PDH conjugate was then desalted at 4° over a G50 column with Tris buffer (0.055 M, pH 8.0) with preservatives.

Example U

Antibodies were produced in conventional manner in response to the conjugate of Example S, and used in the assay together with the enzyme conjugate from Example T.

## Claims

**1.** A method for preparing a serum sample for determination of a tricyclic antidepressant drug in an assay, which method comprises:

(a) adding said serum sample to a chromatographic column containing alkylated silica gel;

(b) washing the column with a wash solution comprising from about 15 to 50 volume percent of an organic solvent of from 1 to 6 carbon atoms and from 1 to 5 heteroatoms selected from the group consisting of oxygen, nitrogen, and sulfur and from about 50 to 85 volume percent of an aqueous buffered medium having a pH of about from 3.5 to 5.0, in an amount sufficient to substantially remove metabolites of the tricyclic antidepressant drug from said column but insufficient to remove the tricyclic antidepressant drug from the column; and

(c) eluting the tricyclic antidepressant drug with an elution solution comprising from about 25 to 100 volume percent of an organic solvent of from 1 to 6 carbon atoms and 1 to 5 heteroatoms selected from the group consisting of oxygen, nitrogen, and sulfur and from about 0 to 75 volume percent of an aqueous buffered medium having a pH of from about 6 to 8 in an amount sufficient to elute a substantial portion of the tricyclic antidepressant drug from the column.

**2.** The method of Claim 1 wherein the silica gel is alkylated with alkyl Groups of from 1 to 6 carbon atoms.

**3.** The method of Claim 1 or Claim 2 therein said alkyl group is ethyl.

**4.** The method of any one of the preceding claims, wherein said organic solvent is an alkylnitrile.

**5.** The method of Claim 4 wherein said organic solvent is acetonitrile.

**6.** The method of any one of the preceding claims, wherein said aqueous buffered medium in Step b contains a metal salt of a carboxylic acid.

**7.** The method of Claim 6 wherein said metal salt is sodium acetate.

**8.** The method of any one of the preceding claims, wherein said aqueous buffered medium in Step b additionally contains an alkyl sulfonate of from 5 to 7 carbon atoms.

**9.** The method of Claim 8 wherein said alkyl sulfonate is heptane sulfonate.

**10.** The method of any one of the preceding claims, wherein the elution solution in Step c comprises two different organic solvents in the ratio of about 1:1 to 1:2.

**11.** The method of Claim 10 wherein the organic solvents are an alkylnitrile and an alcohol.

**12.** The method of Claim 10 wherein the organic solvents are acetonitrile and methanol.

**13.** The method of any one of the preceding claims, wherein the aqueous buffered medium in Step c is an aqueous phosphate buffer.

**14.** The method of Claim 1 wherein said tricyclic antidepressant drug is selected from amitriptyline, impramine, desmethylimipramine, doxepin and desmethyldoxepin.

**15.** A kit for purifying a serum sample to give a tricyclic antidepressant drug sample for use in an assay for said drug, which kit comprises -
(a) a column containing alkylated silica gel,
(b) a wash solution comprising from about 15 to 50 volume percent of an organic solvent of from 1 to 6 carbon atoms and 1 to 5 heteroatoms selected from the group consisting of oxygen, nitrogen, and sulfur and from about 50 to 85 volume percent of an aqueous buffered medium having a pH of about from 3.5 to 5.0; and
(c) an eluent solution comprising about from 25 to 100 volume percent of an organic solvent of from 1 to 6 carbon atoms and 1 to 5 heteroatoms selected from the group consisting of oxygen, nitrogen, and sulfur, and about from 0 to 75 volume percent of an aqueous buffered medium having a pH of about from 6 to 8.

**16.** A tricyclic antidepressant drug sample obtained from a serum sample by the method of any one of Claims 1 to 14.

## Revendications

**1.** Procédé de préparation d'un échantillon de sérum pour le dosage d'un médicament antidépresseur tricyclique lors d'une analyse, procédé qui consiste :
(a) à introduire ledit échantillon de sérum dans une colonne chromatographique contenant un gel de silice alkylé ;
(b) à laver la colonne avec une solution de lavage comprenant environ 15 à 50 % en volume d'un solvant organique ayant 1 à 6 atomes de carbone et 1 à 5 hétéro-atomes choisis dans le groupe comprenant l'oxygène, l'azote et le soufre, et environ 50 à 85 % en volume d'un milieu aqueux tamponné ayant un pH d'environ 3,5 à 5,0, en une quantité suffisante pour chasser pratiquement en totalité les métabolites du médicament antidépresseur tricyclique de ladite colonne, mais insuffisante pour chasser le médicament antidépresseur tricyclique de la colonne ; et
(c) à éluer le médicament antidépresseur tricyclique avec une solution d'élution comprenant environ 25 à 100 % en volume d'un solvant organique ayant 1 à 6 atomes de carbone et 1 à 5 hétéro-atomes choisis dans le groupe comprenant l'oxygène, l'azote et le soufre, et environ 0 à 75 % en volume d'un milieu aqueux tamponné ayant un pH d'environ 6 à 8, en une quantité suffisante pour éluer une forte proportion du médicament antidépresseur tricyclique de la colonne.

2. Procédé suivant la revendication 1, dans lequel le gel de silice est alkylé avec des groupes alkyle ayant 1 à 6 atomes de carbone.

3. Procédé suivant la revendication 1 ou la revendication 2, dans lequel le groupe alkyle est un groupe éthyle.

4. Procédé suivant l'une quelconque des revendications précédentes, dans lequel le solvant organique est un alkylnitrile.

5. Procédé suivant la revendication 4, dans lequel le solvant organique est l'acétonitrile.

6. Procédé suivant l'une quelconque des revendications précédentes, dans lequel le milieu aqueux tamponné utilisé dans l'étape b contient un sel métallique d'un acide carboxylique.

7. Procédé suivant la revendication 6, dans lequel le sel métallique est l'acétate de sodium.

8. Procédé suivant l'une quelconque des revendications précédentes, dans lequel le milieu aqueux tamponné utilisé dans l'étape b contient en outre un sulfonate d'alkyle ayant 5 à 7 atomes de carbone.

9. Procédé suivant la revendication 8, dans lequel le sulfonate d'alkyle est le sulfonate d'heptane.

10. Procédé suivant l'une quelconque des revendications précédentes, dans lequel la solution d'élution utilisée dans l'étape c comprend deux solvants organiques différents, en un rapport d'environ 1:1 à 1:2.

11. Procédé suivant la revendication 10, dans lequel les solvants organiques sont un alkylnitrile et un alcool.

12. Procédé suivant la revendication 10, dans lequel les solvants organiques sont l'acétonitrile et le méthanol.

13. Procédé suivant l'une quelconque des revendications précédentes, dans lequel le milieu aqueux tamponné utilisé dans l'étape c est un tampon aqueux au phosphate.

14. Procédé suivant la revendication 1, dans lequel le médicament antidépresseur tricyclique est choisi entre l'amitriptyline, l'imipramine, la desméthylimipramine, la doxépine et la desméthyldoxépine.

15. Kit destiné à la purification d'un échantillon de sérum pour obtenir un échantillon de médicament antidépresseur tricyclique destiné à être utilisé dans une analyse dudit médicament, kit qui comprend
(a) une colonne contenant un gel de silice alkylé,
(b) une solution de lavage comprenant environ 15 à 50 % en volume d'un solvant organique ayant 1 à 6 atomes de carbone et 1 à 5 hétéro-atomes choisis dans le groupe comprenant l'oxygène, l'azote et le soufre, et environ 50 à 85 % en volume d'un milieu aqueux tamponné ayant un pH d'environ 3,5 à 5,0 ; et
(c) une solution d'élution comprenant environ 25 à 100 % en volume d'un solvant organique ayant 1 à 6 atomes de carbone et 1 à 5 hétéro-atomes choisis dans le groupe comprenant l'oxygène, l'azote et le soufre, et environ 0 à 75 % en volume d'un milieu aqueux tamponné ayant un pH d'environ 6 à 8.

16. Echantillon de médicament antidépresseur tricyclique obtenu à partir d'un échantillon de sérum par le procédé suivant l'une quelconque des revendications 1 à 14.

**Patentansprüche**

1. Verfahren zur Herstellung einer Serumprobe für die Bestimmung eines tricyclischen Antidepressivums in einem Assay, welches Verfahren umfaßt:
(a) Aufgeben der Serumprobe auf eine Chromatographiesäule, die alkyliertes Silicagel enthält;
(b) Waschen der Säule mit einer Waschlösung, die etwa 15 bis 50 Vol.-% eines Organischen Lösungsmittels mit 1 bis 6 Kohlenstoffatomen und 1 bis 5 Heteroatomen, ausgewählt aus der aus

Sauerstoff, Stickstoff und Schwefel bestehenden Gruppe, und etwa 50 bis 85 Vol.-% eines wäßrigen gepufferten Mediums mit einem pH-Wert von etwa 3,5 bis 5,0 umfaßt, in einer Menge, die ausreicht, Metabolite des tricyclischen Ahtidepressivums im wesentlichen von der Säule zu entfernen, aber nicht ausreicht, um das tricyclische Antidepressivum von der Säule zu entfernen; und

(c) Eluieren des tricyclischen Antidepressivums mit einer Elutionslösung, die etwa 25 bis 100 Vol.-% eines organischen Lösungsmittels mit 1 bis 6 Kohlenstoffatomen und 1 bis 5 Heteroatomen, ausgewählt aus der aus Sauerstoff, Stickstoff und Schwefel bestehenden Gruppe, und etwa 0 bis 75 Vol.-% eines wäßrigen gepufferten Mediums mit einem pH-Wert von etwa 6 bis 8 umfaßt, in einer Menge, die ausreicht, um einen wesentlichen Teil des tricyclischen Antidepressivums von der Säule zu eluieren.

**2.** Verfahren nach Anspruch 1, worin, das Silicagel mit Alkylgruppen mit 1 bis 6 Kohlenstoffatomen alkyliert ist.

**3.** Verfahren nach Anspruch 1 oder 2, worin die Alkylgruppe Ethyl ist.

**4.** Verfahren nach irgendeinem der vorhergehenden Ansprüche, worin das organische Lösungsmittel ein Alkylnitril ist.

**5.** Verfahren nach Anspruch 4, Worin das organische Lösungsmittel Acetonitril ist.

**6.** Verfahren nach irgendeinem der vorhergehenden Ansprüche, worin das wäßrige gepufferte Medium in Schritt (b) ein Metallsalz einer Carbonsäure enthält.

**7.** Verfahren nach Anspruch 6, worin das Metallsalz Natriumacetat ist.

**8.** Verfahren nach irgendeinem der vorhergehenden Ansprüche, worin das wäßrige gepufferte Medium in Schritt (b) weiterhin ein Alkylsulfonat mit 5 bis 7 Kohlenstoffatomen enthält.

**9.** Verfahren nach Anspruch 8, worin das Alkylsulfonat ein Heptansulfonat ist.

**10.** Verfahren nach irgendeinem der vorhergehenden Ansprüche, worin die Elutionslösung in Schritt (c) zwei verschiedene organische Lösungsmittel im Verhältnis von etwa 1:1 bis 1:2 umfaßt.

**11.** Verfahren nach Anspruch 10, worin die organischen Lösungsmittel ein Alkylnitril und ein Alkohol sind.

**12.** Verfahren nach Anspruch 10, worin die organischen Lösungsmittel Acetonitril und Methanol sind.

**13.** Verfahren nach irgendeinem der vorhergehenden Ansprüche, worin das wäßrige gepufferte Medium in Schritt (c) ein wäßriger Phosphatpuffer ist.

**14.** Verfahren nach Anspruch 1, worin das tricyclische Antidepressivum aus Amitriptylin, Imipramin, Desmethylimipramin, Doxepin und Desmethyldoxepin ausgewählt ist.

**15.** Kit zur Reinigung einer Serumprobe, um eine Probe eines tricyclischen Antidepressivums zur Verwendung in einem Assay für dieses Arzneimittel bereitzustellen, welches Kit umfaßt:

(a) eine Säule, die alkyliertes Silicagel enthält;

(b) eine Waschlösung, die etwa 15 bis 50 Vol.-% eines organischen Lösungsmittels mit 1 bis 6 Kohlenstoffatomen und 1 bis 5 Heteroatomen, die aus der aus Sauerstoff, Stickstoff und Schwefel bestehenden Gruppe ausgewählt sind, und etwa 50 bis 85 Vol.-% eines wäßrigen gepufferten Mediums mit einem pH-Wert von etwa 3,5 bis 5,0 % umfaßt; und

(c) eine Elutionslösung, die etwa 25 bis 100 Vol.-% eines organischen Lösungsmittels mit 1 bis 6 Kohlenstoffatomen und 1 bis 5 Heteroatomen, die aus der aus Sauerstoff, Stickstoff und Schwefel bestehenden Gruppe ausgewählt sind, und etwa 0 bis 75 Vol.-% eines wäßrigen gepufferten Mediums mit einem pH-Wert von etwa 6 bis 8 umfaßt.

**16.** Probe eines tricyclischen Antidepressivums, die aus einer Serumprobe durch das Verfahren nach irgendeinem der Ansprüche 1 bis 14 erhalten wurde.